# EUROPÄISCHE PATENTANMELDUNG

(11) **EP 3 401 816 A1**
(43) Veröffentlichungstag der Anmeldung: **14.11.2018**
(21) Anmeldenummer: 17170584.1
(22) Anmeldetag: 11.05.2017
(51) Int. Cl.: G06F 19/00

(54) **DYNAMISCHE ERSTELLUNG VON ÜBERSICHTSNACHRICHTEN IM GESUNDHEITSWESEN**

(71) Anmelder: Siemens Healthcare GmbH, 91052 Erlangen (DE)
(72) Erfinder: MANGESIUS, Patrick, 6020 Innsbruck (AT)

(57) **Zusammenfassung**

Die vorliegende Erfindung betrifft ein Verfahren, ein System und unterschiedliche Knoten zum Bereitstellen einer Übersichtsnachricht (uen) für einen Patienten. In der Übersichtsnachricht sind alle relevanten Datensätze zur bisherigen Behandlung des Patienten zusammengestellt und sie enthält Verweise zum Zugriff auf die jeweiligen Dokumente, in denen die Bestandteile der Übersichtsnachricht (uen) enthalten sind und für die Zugriffsberechtigungen validiert werden konnten.

## Beschreibung

Die vorliegende Erfindung betrifft Verfahren und ein System zum Erstellen einer Übersichtsnachricht für einen Patienten auf Basis einer standardisierten Plattform.

Die medizinischen oder gesundheitsbezogenen Daten eines Patienten werden in unterschiedlichen Einrichtungen, wie Kliniken und Instituten erzeugt. Sie liegen somit in verteilter Form (lokal) vor. Die jeweiligen Einrichtungen sind üblicherweise vernetzt. Die Daten werden zunehmend digital erfasst oder zumindest in digitaler Form abgelegt.

Zur Verbesserung einer Behandlungsqualität für den Patienten und zur Erhöhung der Sicherheit der Behandlung ist es wünschenswert, dass der Austausch von Daten zwischen den unterschiedlichen verteilten Einrichtungen verbessert wird. Dies dient auch dazu, unnötige Mehrfachuntersuchungen zu vermeiden und insgesamt die Kosten im Gesundheitssektor zu senken.

Eine zentrale Speicherung von medizinischen Daten ist in vielen Ländern aufgrund von Datenschutzbestimmungen nicht möglich. Dies bedeutet, dass die medizinischen Daten, wie z.B. Anamnesedaten, bisherige Befunde oder Berichte, erzeugte Bilddaten etc. nicht oder zumindest nicht ungesichert die Sphäre der jeweiligen Einrichtung verlassen dürfen, auf der sie erfasst worden sind; die Daten verbleiben somit lokal und liegen deshalb verteilt und dezentral vor.

Ein weiteres Problem im Stand der Technik besteht darin, dass die Anzahl von Daten (gespeicherten medizinischen Daten) zunimmt. Die Auswahl von relevanten Daten für den jeweiligen Anwendungsfall stellt somit eine Herausforderung dar. Selbst wenn die Daten nicht in dezentralen Datentöpfen vorliegen würden, sondern zentral, dann wäre das Extrahieren von relevanten Informationen aus diesen sehr umfangreichen Datenvolumina eine rechenlast- und ressourcen-intensive Aufgabe, da eine Vielzahl von unterschiedlichen speicherplatzintensiven Dateien durchsucht werden muss. Die lokalen Einrichtungen sind jedoch nicht immer mit den notwendigen infrastrukturellen (informationstechnologischen) Mitteln ausgestattet.

Erschwerend ist des Weiteren zu berücksichtigen, dass die Anforderungen an die Auswahl von Daten sehr unterschiedlich sein können. Zum einen gilt es unterschiedliche Berechtigungen der Teilnehmer (z.B. in der Rolle als Arzt, Patient, Personal etc.), die zudem über die Zeit variieren können, zu berücksichtigen und zum anderen existieren unterschiedliche Anwendungen und medizinische Fragestellungen. So macht es beispielsweise einen Unterschied, ob die Daten für eine medizinische Studie oder für eine konkrete Diagnose eines Patienten erfasst werden sollen, wobei Letzteres in der Regel einen höheren Detaillierungsgrad der Daten erfordert.

Wünschenswert ist es somit, Gesundheitsdaten auch über die Grenzen der jeweiligen Einrichtung hinaus verfügbar zu machen und dabei die oben erwähnten Restriktionen und Vorgaben zu erfüllen.

Im Stand der Technik wird zum Datenaustausch von elektronischen Gesundheitsdaten in EHR-Systemen (EHR - Electronic Health Record) zwischen den verteilten Institutionen üblicherweise auf Standards zurückgegriffen. IHE (IHE - Integrating the Healthcare Enterprises) ist z.B. eine internationale Initiative von Anwendern und Herstellern mit dem Ziel, den Datenaustausch zwischen IT-Systemen im Gesundheitswesen zu standardisieren und zu harmonisieren. XDS ist ein Beispiel eines einrichtungsübergreifenden Dokumentenaustauschsystems (Cross-Enterprise Document Sharing). In IHE XDS basierten Umgebungen sind drei unterschiedliche Akteure vorgesehen:
1. Eine XDS Quelle, die die Daten erzeugt (z.B. ein bildgebendes System oder eine Messvorrichtung),
2. eine XDS Repository, die zur lokalen persistenten Speicherung der erfassten Daten und deren Registrierung im Repository dient und
3. eine zentralen Registry.

Die Registry ist verantwortlich für die Speicherung von Metadaten über diese erzeugten Daten bzw. Dokumente, so dass die für die Behandlung eines Patienten relevanten Dokumente leicht gefunden, ausgewählt und abgerufen werden können, unabhängig von der Repository, also unabhängig von dem Speicherort (in der klinischen Einrichtung), auf dem sie tatsächlich gespeichert sind. Dazu enthalten die Metadaten einen Zeiger (pointer, link, Verweis) auf den Speicherort des Dokumentes in dem Repository der jeweiligen lokalen Einrichtung (z.B. in einer informationstechnologischen Einheit der Klinik etc.). Es können mehrere lokale Repositories von einer oder von unterschiedlichen Einrichtung(en) an eine Registry angebunden werden. In diesem Standard ist es ein wichtiger Aspekt, dass die Registry keinen Zugriff auf die in der Repository abgelegten Dokumente hat, sie verfügt nur über Zeiger auf deren Speicherort. Diese Vorkehrung dient dazu, die Sicherheitsbestimmungen einzuhalten, nämlich um sicherzustellen, dass die sicherheitskritischen, medizinischen Daten die jeweilige Einrichtung nicht verlassen. Ein Client bzw. eine Datensenke kann mit vorhandener Zugriffsberechtigung auf die Dokumente in dem Repository zugreifen.

Wie oben bereits erwähnt, besteht aufgrund der hohen Datenmenge die Herausforderung, relevante Daten bereitstellen zu können. Dazu können ein Übersichtsdokument bzw. eine patienten-spezifische Zusammenfassungsdatei dienen. Im HL7 Standard (Health Level 7) für den Austausch von Daten zwischen Organisationen im Gesundheitswesen ist dazu bereits ein sogenanntes C-CDA Dokument definiert. "CDA" steht für ein "Clinical Document Architecture" und das vorangestellte "C-" für den Begriff "consolidated". CDA ist ein XML-basiertes Schema für alle klinischen Dokumente im Gesundheitssektor. Ein Übersichtsdokument ist somit in diesem Standard bereits vorgesehen. Die Erzeugung eines solchen Übersichtsdokumentes ist im Stand der Technik jedoch sehr aufwändig, da für die Extraktion jeweils eines Bestandteils des Übersichtsdokumentes das jeweils korrespondierende Basisdokument geöffnet und durchsucht werden muss, in dem der Bestandteil enthalten ist.

Aufgrund der eingangs geschilderten technischen Anforderungen (Sicherheit, Datenschutz etc.) war es in IHE XDS basierten Systemen notwendig, jedes einzelne Dokument zu öffnen, wenn daraus Daten extrahiert werden sollten, die für eine Übersichtsdarstellung relevant sind. Sollte beispielsweise eine Übersichtsdatei für eine spezielle Anwendung Angaben über das Gewicht und das Alter des Patienten umfassen, so ist es bei den bisherigen Systemen notwendig, eine erste Datei zu öffnen, in der Angaben zum Gewicht abgelegt sind und diese Daten zu übernehmen und eine zweite Datei zu öffnen, in der Angaben über das Alter hinterlegt sind. Für einen Fachmann liegt es auf der Hand, dass dieses Vorgehen, selbst wenn es automatisiert wird, sehr rechen- und zeit-intensiv ist und daher einen hohen Ressourcenaufwand bedeutet.

Ausgehend von dieser Problemstellung liegt der vorliegenden Erfindung die Aufgabe zugrunde, einen Weg aufzuzeigen, um den Datenaustausch im Gesundheitsbereich zu verbessern unter Einhaltung der oben beschriebenen Einschränkungen und Anforderungen. Insbesondere soll es möglich sein, auf eine Anfrage eines Clients in dynamischer Weise ein Übersichtsdokument aus einer Vielzahl von dezentral gespeicherten Datenquellen zu erzeugen und dabei die Zugriffsberechtigungen der jeweiligen Clients mit veränderlichen Berechtigungsregeln einzuhalten.

Diese Aufgabe wird jeweils durch einen Gegenstand nach den unabhängigen Ansprüchen gelöst. Vorteilhafte Ausführungsformen sind Gegenstand der abhängigen Ansprüche, der Beschreibung und der Zeichnungen.

Im Gesundheitsbereich werden eine Vielzahl von Daten erzeugt und müssen verwaltet werden. Für medizinische Fragestellungen ist ein gezielter Zugriff auf relevante Daten wichtig, die aus verteilten Datenquellen berechnet werden. Ein Aspekt der Erfindung bezieht sich darauf, diese relevanten Daten in Form einer Übersichtsnachricht bzw. in Form einer Patienten-Zusammenfassungsdatei zu erzeugen.

Im Folgenden werden die im Rahmen dieser Anmeldung verwendeten Begrifflichkeiten definiert.

Die Registry ist ein digitaler Speicher, der zentral vorgehalten wird. Er kann als Cloud-Speicher über entsprechende Netzwerkzugriffe angesprochen werden. Vorzugsweise handelt es sich um eine XDS-Registry. Die Registry dient als Dokumenten-Metadaten-Index, also als Metadaten-Index für die erfassten Datensätze.

Das Repository ist ein digitaler Speicher, z.B. in Form einer Festplatte oder eines RAID-Systems, der lokal vorgehalten ist. Er dient zur lokalen Speicherung der erfassten Datensätze bzw. Dokumente. Vorzugsweise ist er als XDS-Repository ausgebildet.

Der Begriff "Datensatz" kann auch im Sinne von "Dokument" verstanden werden. Zunächst werden auf einer Datenquelle (technische Einrichtung im Gesundheitswesen), wie z.B. einem bildgebenden Gerät oder einem Messgerät oder auf einem sonstigen Gesundheitsapparat, Datensätze erfasst. Diese werden in einem Dokument gespeichert und können weiterverarbeitet werden. Das Dokument kann eine bestimmte Struktur aufweisen, die eine weitere automatische Verarbeitung erleichtert. Die Struktur kann auf einem Standard basieren. Vorzugsweise basiert die Struktur auf einem IHE-XDS Standard und es handelt sich um ein CDAformatiertes Dokument (CDA: Clinical Document Architecture). Andere Ausführungsformen der Erfindung verwenden ein anderes Format.

Der Brokerservice ist ein Dienst, der auf einem computerbasierten Knoten mit einer Verarbeitungseinheit ausgeführt wird. Dazu wird ein Brokerknoten bereitgestellt, der zur Ausführung des Brokerservices bestimmt ist. Der Brokerservice kann in Software implementiert sein. Der Brokerservice wird als zentraler Dienst bereitgestellt. Vorzugsweise ist der Brokerservice ausgebildet, um in eine IHE-XDS Plattform integriert zu werden.

Der Extraktionsservice ist ein Dienst, der auf einem computerbasierten Knoten mit einer Recheneinheit ausgeführt wird. Dazu wird ein Extraktionsknoten bereitgestellt, der zur Ausführung des Extraktionsservices bestimmt ist. Der Extraktionsservice kann in Software implementiert sein. Der Extraktionsservice wird als lokaler Dienst (z.B. in einer Klinik oder einem Client zugeordnet) bereitgestellt. Vorzugsweise ist der Extraktionsservice ausgebildet, um in eine IHE-XDS Plattform integriert zu werden.

Ein Client ist eine elektronische Instanz, wie z.B. eine computerbasierte Einheit, wie ein Rechner, ein mobiles Gerät, ein Tablet ein Smartphone oder eine Workstation. Der Client kann von unterschiedlichen Anwendern in unterschiedlichen Rollen (z.B. Arzt, Schwester, Pflegekraft) bedient werden. Je nach Rolle, können unterschiedliche Zugriffsrechte auf die Daten vergeben werden. Dies kann in einem Zugriffsrechteverwaltungssystem implementiert sein. Der Client kann als Sender und als Empfänger von Datensätzen fungieren. Der Client kann dazu dienen, die Übersichtsnachricht zu erfragen oder Datensätze zu erzeugen (z.B. einen Arztbericht).

Die Übersichtsnachricht ist ein elektronischer Datensatz, der als Zusammenfassung für die bisherige Behandlung des Patienten oder - bei komplexen Verläufen - von Abschnitten daraus dienen soll. Ein von einer Datenquelle (z.B. einem medizinischen Gerät) übermitteltes strukturiertes Dokument (z.B. CDA Dokument) kann ein oder mehrere Observationsnachrichten bzw. Extraktionsnachrichten an den Service (insbesondere zunächst an den lokalen Extraktionsservice und dann an den zentralen Brokerservice) schicken. Wenn z.B. ein Dokument Informationen über Allergien und Gewicht umfasst, kann daraus auch eine erste Nachricht mit <Allergien> und eine zweite Nachricht mit <Gewicht> erzeugt werden, je nach der gewählten Implementierung. Die Übersichtsnachricht dient zur übersichtsartigen Repräsentation von ausgewerteten patienten-spezifischen Datensätzen, Daten und/oder Parameterwerten oder komplexeren Zusammenstellungen von Daten in Form von Berichten, Befunden oder sonstigen medizinischen Ergebnissen, die in der Übersichtsnachricht aggregiert und zusammengefasst sind. Die Übersichtsnachricht enthält neben den medizinischen oder gesundheitsbezogenen Daten einen den Patienten eindeutig identifizierenden Hinweis (den Patienten-Identifikator) und einen eindeutig identifizierenden Hinweis auf das jeweilige Dokument, in dem der Bestandteil bzw. Parameter der Übersichtsnachricht enthalten ist (Dokumenten-Identifikator). Die einzelnen Bestandteile der Übersichtsnachricht liegen in der Regel verteilt auf unterschiedlichen Systemen und in unterschiedlichen Formaten vor. Sie dürfen auch das jeweilige Netzwerk nicht (ungeschützt) verlassen. Welche Bestandteile in der Übersichtsnachricht enthalten sein sollen, kann vorzugsweise in einer Vorbereitungsphase konfiguriert werden. So können beispielsweise in einer Ausführungsform der Erfindung das Gewicht, das Alter, vorhandene Allergien und die aktuelle Medikation des Patienten als Bestandteile für die Übersichtsnachricht bestimmt werden. Die Daten werden vorzugsweise so strukturiert, dass sie effizient verarbeitet und eingelesen werden können. Sie können z.B. als aus den jeweiligen Bestandteilen zusammengesetzter String formatiert sein.

Gemäß einer bevorzugten Ausführungsform der Erfindung kommt der Übersichtsnachricht eine doppelte Funktionalität zu. Zum einen enthält sie die jeweils gewünschten, vorkonfigurierbaren Bestandteile (z.B. Alter, Gewicht etc.) und zum anderen enthält sie einen Verweis (Link) auf das jeweilige Dokument, in dem der jeweilige Bestandteil enthalten ist (also insgesamt eine Menge von Verweisen auf eine Menge von verteilt gespeicherten Dokumenten). Bei Bedarf kann ein Client, an den die Übersichtsnachricht mittels des hier vorgeschlagenen Verfahrens oder Systems ausgeliefert wird, über den Link dann auf weitere, detailliertere Informationen zugreifen. Dies kann sozusagen optional und in einem zweiten Schritt ausgeführt werden. Die Übersichtnachricht ermöglicht ihm allerdings zunächst in einem ersten Schritt eine patienten-spezifische Zusammenfassung von Parameterwerten oder sonstigen Daten einzusehen, ohne dass er die einzelnen Dokumente, in denen die Parameter enthalten sind, öffnen muss. Die Zugriffszeiten und die Zeit zur Suche nach medizinischen Details in umfangreichen Dokumentenansammlungen können damit vorteilhafterweise deutlich gesenkt werden.

Die Extraktionsnachricht ist ein elektronischer Datensatz, der aus den erfassten medizinischen Daten der Datenquelle extrahiert wird. Die Extraktion wird durch einen spezifischen Service durchgeführt, nämlich den Extraktionsservice bzw. Extraktionsdienst. Der Extraktionsdienst kann ebenfalls in der Vorbereitungsphase dahingehend konfiguriert werden, welche Elemente er aus dem erfassten Datensatz extrahieren soll. Die Extraktionsnachricht umfasst bei einfachen numerischen Typen (Alter, Gewicht etc.) den Typ (z.B. Gewicht) und den jeweiligen Wert (z.B. 75kg). Bei komplexen Typen (z.B. MRT Aufnahmen) kann nur eine Typangabe extrahiert werden (Angabe: Bild oder MRT-Bild oder eine im Bild repräsentierte anatomische Region).

Die jeweiligen Knoten (z.B. Extraktionsknoten, Brokerknoten) sind computerbasierte Hardwareeinheiten. Ein Knoten kann als Netzwerkknoten ausgebildet sein. Es kann sich insbesondere um ein Gerät handeln, das Vermittlungsfunktionen übernimmt, aber ebenso um eine Vorrichtung, die am Netzrand als Interworking Unit arbeitet. Ein Knoten kann ein Controller, Knotenrechner, Terminal, Drucker, Plattenlaufwerk, Minicomputer, Gateway oder eine andere Einrichtung sein. Ein Knoten kann Datenkanäle vervielfachen, teilen und statistisch zuordnen sowie mittels dynamischer Zuordnung Datenübertragungskanäle vermitteln.

Bei den beschriebenen Schnittstellen (Interfaces) handelt es sich um Bestandteile des technischen Systems, die der Kommunikation dienen. Die Schnittstelle kann als Netzwerkschnittstelle (z.B. WLAN oder eine andere Funkschnittstelle) ausgebildet sein. Es können auch mehrere Schnittstellen pro Netzwerksegment bzw. Kommunikationsgerät vorgesehen sein, wie z.B. bei Verwendung einer Bridge mit mindestens zwei Netzwerkschnittstellen (wobei die Bridge/Brücke dafür sorgt, dass Signale, Protokolle und/oder Übertragungssysteme konvertiert werden, falls die kommunizierenden Systeme keine gemeinsame Schnittstelle haben) oder bei Verwendung von Hubs und Switches oder Routern. Auf einer anderen ISO/OSI-Ebene kann die Schnittstelle auch als Softwareschnittstelle ausgebildet sein und die jeweiligen Applikationen oder Betriebssystemprogramm datentechnisch verbinden. Es kann sich bei den Schnittstellen somit um softwareseitige Datenschnittstellen handeln, die logische Verbindungen in einem Softwaresystem bereitstellen. Sie ermöglichen und regeln den Austausch von Kommandos und/oder Daten zwischen verschiedenen Prozessen und Komponenten. Die Schnittstelle kann zur Interprozesskommunikation und/oder als Schnittstelle für Programmkomponenten bereitgestellt werden. Insbesondere werden die drahtlosen Schnittstellen der bekannten Netzwerkprotokolle, wie TCP, HTTP usw. als Schnittstellen verstanden.

Bei der Verarbeitungseinheit und der Recheneinheit handelt es sich um computer-basierte Instanzen, die zur Ausführung von Computerprogrammen dienen. Es kann sich insbesondere um einen Prozessor, um einen Microcontroller und/oder um einen Microprozessor handeln. Die Verarbeitungs- und Recheneinheit kann als zentrale Verarbeitungseinheit (central processing unit, CPU) ausgebildet sein und dazu dienen Befehle eines Programms abzuarbeiten. Sie kann ein Rechenwerk und ein Steuerwerk und Kommunikationsschnittstellen umfassen.

Gemäß einem ersten Aspekt wird die Aufgabe gelöst durch ein Verfahren zum Betreiben eines Extraktionsservices zum Erzeugen einer (elektronischen) Extraktionsnachricht für einen Patienten auf Basis einer standardisierten Plattform, umfassend folgende Verfahrensschritte:
- Einlesen eines Datensatzes von einer lokalen Datenquelle;
- Auswählen von Parametern des eingelesenen Datensatzes zum Erzeugen einer Extraktionsnachricht;
- Versenden der Extraktionsnachricht an einen zentralen Brokerservice.

Gemäß dem IHE XDS Standard ist vorgesehen, dass der eingelesene Datensatz an ein lokales Repository zur Speicherung versendet wird. Das Repository ist ausgebildet, Metadaten von dem (von der Datenquelle empfangenen) Datensatz an eine zentrale Registry zum Zwecke der Registrierung zu senden. Auf der Registry liegen somit nur Metadaten zu den auf der Datenquelle erfassten Daten. Die eigentlichen (Binär-) Daten sind lediglich lokal in dem Repository gespeichert. Gemäß einer bevorzugten Ausführungsform der Erfindung setzt der Ablauf auf diesem standardisierten Vorgehen auf. Ein wichtiger Vorteil ist deshalb darin zu sehen, dass der standardgemäße Ablauf nicht verändert oder beeinflusst werden muss, um die Vorteile der Erfindung zu erreichen. Die Krankenhäuser noch die angeschlossenen Clients müssen keine neue Hardware (bzw. Software) kaufen. Es ist lediglich erforderlich zwei Services (einen lokalen Extraktionsservice und einen zentralen Brokerservice) zur Verfügung zu stellen. Diese können vorteilhafterweise auf bestehende Knoten integriert und implementiert sein. Prinzipiell kann das hier vorgestellte Konzept und die Erfindung auch mit anderen Standards und ebenfalls mit proprietären Standards und ebenfalls mit nicht-standardkonformen Systeme zur Anwendung kommen und nicht ausschließlich mit XDS zusammenspielen.

In einer vorteilhaften Ausführungsform des Verfahrens ist es vorkonfigurierbar (in einer Konfigurationsphase des Verfahrens), welche der Parameter aus dem eingelesenen Datensatz zu extrahieren sind. Das bedeutet, dass sich das Verfahren in eine Ausführungsphase und in eine vorgelagerte Konfigurationsphase einteilen lässt und, dass in der Konfigurationsphase eingestellt werden kann, welche Parameter grundsätzlich extrahiert werden sollen. Diese werden als extraktionsfähig gekennzeichnet.

Gemäß einer weiteren vorteilhaften Ausführungsform des Verfahrens umfasst die Extraktionsnachricht zumindest folgende Elemente:
- Einen Patienten-Identifikator, der den jeweiligen Patienten eindeutig kennzeichnet;
- einen Dokumenten-Identifikator, der einen Speicherort von Metadaten zu dem eingelesenen Datensatz eindeutig referenziert, aus dem (Datensatz) die Daten extrahiert wurden;
- eine konfigurierbare Auswahl von extrahierten Parametern, insbesondere unter Angabe eines Parametertyps (z.B. Gewicht, Alter, Allergie etc.) und dessen Wert (z.B. 67 kg, 35, Heuschnupfen). Vorzugsweise wird dabei ein Code verwendet, der einen Parametertyp eindeutig kennzeichnet. In einer bevorzugten Ausführungsform der Erfindung ist ein Parameter somit durch die Angabe eines Parametertyps und des entsprechenden Wertes gekennzeichnet.

Es ist zu beachten, dass der Begriff "Parameter" in unterschiedlichen Bezügen bzw. Instanzen in der Anmeldung verwendet wird. So gibt es Parameter, die in der angefragten Übersichtsnachricht, in der erstellten Übersichtsnachricht, in der Extraktionsnachricht und in aggregierten Extraktionsnachrichten enthalten sind. Der Parameter (unter Angabe von Parametertyp und Parameterwert) referenziert damit die jeweils zugeordneten Datensätze und dient in technischer Hinsicht als Index. Der Index erlaubt es, die Speicherposition des jeweils angefragten Datensatzes innerhalb des Speichermediums schnell bestimmen zu können, so dass nur eine Teilmenge der gespeicherten Daten durchsucht werden muss. Dies hat den technischen Vorteil, dass eine schnelle Suche in großen Datenmengen effizient ausgeführt werden kann und dabei gleichzeitig die Sicherheitsbestimmungen und Zugriffsregelungen einhalten zu können.

Wenn beispielsweise die angefragte Übersichtsnachricht den Parameter "Blutgruppe" umfassen soll, so wird der Parameter "Blutgruppe" zunächst aus der Übersichtsnachricht ermittelt, um daraufhin damit einen Zugriff auf die Datenbank auszuführen, um diejenigen Extraktionsnachrichten zu sammeln und zusammenzufassen, die den Parameter "Blutgruppe" umfassen. Für die aggregierten Extraktionsnachrichten wird dann unter Zugriff auf die zentrale Registry das Zugriffsrecht des anfragenden Clients auf die "Blutgruppen"-Datensätze ermittelt und nur falls eine Berechtigung validiert werden kann, wird der jeweilige Parameter in die zu erstellende Übersichtsnachricht inkludiert. Liegen z.B. zwei unterschiedliche Datensätze in der Datenbank bereit, die den Parameter "Blutgruppe" umfassen, nämlich ein erster Datensatz, der eine Diagnose (z.B. in Form einer Diagnose "AIDS" unter Bezugnahme auf die Blutgruppe in einem Arztbericht) umfasst und ein zweiter Datensatz, der einen Ausschnitt von Daten eines Impfpasses umfasst, so kann z.B. die Zugriffsberechtigung des Clients für den zweiten Datensatz vorliegen, aber für den ersten Datensatz nicht. In diesem Fall wird nur der zweiten Datensatz in der zu erstellenden Übersichtsnachricht referenziert, da nur für diesen eine Berechtigung vorliegt.

Gemäß einem zweiten Aspekt wird die Aufgabe gelöst durch ein Verfahren zum Betreiben eines zentralen Brokerservices zum Bedienen einer Anfrage eines Clients für eine Übersichtsnachricht für einen Patienten auf Basis einer standardisierten Plattform, umfassend folgende Verfahrensschritte:
- Empfangen der Anfrage des Clients;
- Auflösen der empfangenen Anfrage und Bestimmen eines Client-Identifikators und Ermitteln von Parametern der angefragten Übersichtsnachricht;
- Zugriff auf eine Datenbank, um diejenigen Extraktionsnachrichten zu aggregieren, die den ermittelten Parametern entsprechen;
- Zugriff auf eine zentrale Registry, um auf Basis des bestimmten Client-Identifikators eine existierende Berechtigung des Clients zum Zugriff auf das jeweilige Dokument zu überprüfen, in dem der jeweilige Parameter aller aggregierten Extraktionsnachrichten enthalten ist und bejahendenfalls (also falls die Berechtigung vorhanden ist):
- Einfügen des jeweiligen Parameters in die Übersichtsnachricht und iteratives
- Erstellen der Übersichtsnachricht.

In einer bevorzugten Ausführungsform der Erfindung wird die Übersichtsnachricht dynamisch und nur bei Bedarf auf Anfrage des Clients und insbesondere während des Zugriffs (auf die Datenbank) erzeugt wird. Damit können vorteilhafterweise die Sicherheitsreglementierung eingehalten werden und die zu schützenden Daten werden ausschließlich lokal bzw. vor Ort (der Datenerfassung) persistent gespeichert.

Gemäß einer weiteren bevorzugten Ausführungsform der Erfindung wird die Übersichtsnachricht als vollstrukturiertes Dokument ausgeliefert, und ist insbesondere nach einem CDA-Standard formatiert. Dies erleichtert die Weiterverarbeitung der Daten und die Einbindung der Übersichtsnachricht in andere standardisierte Systeme.

Gemäß einer weiteren bevorzugten Ausführungsform der Erfindung sind in der zentralen Registry Berechtigungsregeln hinterlegt, die auch während Ausführung des Verfahrens veränderlich sind. Die Berechtigungsregeln dienen zur Festlegung der Zugriffsrechte der Clients. Vorteilhafterweise kann mit der getrennten Speicherung der Berechtigungsregeln eine dynamische Anpassung an den Anwendungsfall erzielt werden.

Gemäß einer weiteren bevorzugten Ausführungsform der Erfindung werden beim Erzeugen der Übersichtsnachricht eine Zugriffsberechtigung des anfragenden Clients und/oder Kontextparameter der Anfrage berücksichtigt. Je nach Rolle (z.B. Position des Anwenders mit jeweils unterschiedlicher dedizierter Zugriffsberechtigung) und Kontext (z.B. Verwendungszweck und Hintergrund bzw. Sinn der Anfrage, beispielsweise für eine Diagnose oder für eine Studie) einer Anfrage gelten unterschiedliche Zugriffsrechte auf die jeweiligen Datensätze. Vorteilhafterweise können die Berechtigungsregeln auch während des Betriebs geändert werden, ohne dass ein Neustart des Verfahrens notwendig wird. In der Informatik ist es bekannt, eine Benutzerrolle (oder kurz Rolle) zu definieren. Dabei kann eine Menge von Einzel-Rechten zum Zugriff auf sicherheitskritische Daten und/oder an einer Software zusammengefasst werden. Benutzerrollen werden verwendet, um die Einzel-Rechte der vorgenannten Bereiche nicht für jeden Nutzer einzeln festlegen zu müssen: Statt vielen Benutzern dieselben einzelnen Rechte direkt zuzuweisen, wird eine Benutzerrolle definiert, welche die zuzuweisenden Rechte enthält. Den Benutzern wird dann die entsprechende jeweilige Rolle zugewiesen. Dies erleichtert die Rechteverwaltung des Softwaresystems, da insbesondere bei Änderungen der Rechtestruktur nur die Rechte der Benutzerrolle angepasst werden müssen. Die Rechteverwaltung kann in einem Zugriffskontrollsystem (access control system) implementiert werden. Vorzugsweise ist die Benutzeroberfläche des Clients an seine Benutzerrolle angepasst, d.h., je nachdem über welchen Berechtigungen er verfügt, kann er umfangreichere Anfragen generieren oder nur einfache. Die Benutzerrolle mit den Zugriffsberechtigungen kann in einem mobilen Datenträger bereitgestellt werden, der über eine drahtlose Schnittstelle ausgelesen werden kann (z.B. in Form einer Chipkarte oder eines RFID-Tags).

Gemäß einer weiteren bevorzugten Ausführungsform der Erfindung werden zum Erzeugen der Übersichtsnachricht deren angefragte Parameter nach konfigurierbaren Filterkriterien gefiltert.

Gemäß einer weiteren bevorzugten Ausführungsform der Erfindung umfasst die Übersichtsnachricht einen Link auf das jeweilige Dokument, in dem der/die angefragte(n) Parameter enthalten ist/sind. In der Regel enthält die Übersichtsnachricht eine Menge von Parametern und somit eine Menge von Links, die auf die jeweiligen Dokumente bzw. deren Speicherort verweisen. Wie vorstehend bereits beschrieben, werden in einem vorlagerten Schritt die jeweiligen Zugriffsrechte des Clients auf die Dokumente geprüft und zentral gespeichert (vorzugsweise in der Registry). Dies kann in einer bevorzugten Ausführungsform der Erfindung mittels eines Berechtigungsservices (Access Control System) ausgeführt werden. Falls während des Betriebs ein Zugriffsrecht nicht validiert werden konnte (also kein Zugriff vorliegt), dann wird der Parameter automatisch aus der Übersichtsnachricht entfernt und enthält entsprechend auch keinen Link auf das Dokument.

Gemäß einer weiteren bevorzugten Ausführungsform der Erfindung wird die Übersichtsnachricht nicht in der Registry gespeichert. Sie wird vorzugsweise auf keinem Knoten gespeichert, sondern nur dem Client auf seine Anfrage zur Verfügung gestellt. Damit können die Sicherheit erhöht und ein Missbrauch der Daten vermieden werden.

Gemäß einer weiteren bevorzugten Ausführungsform der Erfindung werden alle Berechnungen zur Erzeugung der Übersichtsnachricht vollständig durch den zentralen Brokerservice ausgeführt. Damit entsteht der wichtige Vorteil, dass die gesamte Rechenlast und der Ressourcenverbrauch sozusagen auf den Server oder in die Cloud ausgelagert werden können. Optional kann auch ein Zugriffskontroll- oder Berechtigungssystem zugegriffen werden.

Gemäß einer weiteren bevorzugten Ausführungsform der Erfindung ist die standardisierte Plattform eine IHE-XDS Plattform.

In einem anderen Aspekt bezieht sich die Erfindung auf einen Extraktionsknoten zum Erzeugen einer Extraktionsnachricht für einen Patienten auf Basis einer standardisierten Plattform, umfassend:
- Eine Extraktionseingangsschnittstelle, die zum Einlesen eines Datensatzes von einer lokalen Datenquelle bestimmt ist;
- Einer Recheneinheit, die zum Auswählen von Parametern des eingelesenen Datensatzes zur Erzeugung einer Extraktionsnachricht bestimmt ist;
- Eine Extraktionsausgangsschnittstelle, die zum Versenden der Extraktionsnachricht an einen zentralen Brokerservice bestimmt ist.

In einem anderen Aspekt bezieht sich die Erfindung auf einen zentralen Brokerknoten, der zur Ausführung eines Brokerservices dient zum Bedienen einer Anfrage eines Clients für eine Übersichtsnachricht für einen Patienten auf Basis einer standardisierten Plattform, umfassend:
- Eine Brokereingangsschnittstelle, die zum Empfangen der Anfrage des Clients dient;
- Einer Brokerextraktionsschnittstelle, die zum Einlesen einer Extraktionsnachricht bestimmt ist;
- Einer Verarbeitungseinheit, die zum Auflösen der empfangenen Anfrage und zum Bestimmen eines Client-Identifikators und zum Ermitteln der Parameter der angefragten Übersichtsnachricht bestimmt ist;
- und wobei die Verarbeitungseinheit weiterhin zum Zugriff auf eine Datenbank bestimmt ist, um diejenigen Extraktionsnachrichten zu aggregieren, die den ermittelten Parametern entsprechen;
- und wobei die Verarbeitungseinheit weiterhin zum Zugriff auf eine zentrale Registry bestimmt ist, um auf Basis des bestimmten Client-Identifikators eine existierende Berechtigung des Clients zum Zugriff auf das jeweilige Dokument zu überprüfen, in dem der jeweilige Parameter aller aggregierten Extraktionsnachrichten enthalten ist und
- wobei die Verarbeitungseinheit bei existierender Berechtigung zum Einfügen des jeweiligen Parameters in die Übersichtsnachricht und zum Erstellen der Übersichtsnachricht bestimmt ist.

In einem anderen Aspekt bezieht sich die Erfindung auf ein System zum Erzeugen einer Übersichtsnachricht für einen Patienten auf Basis einer standardisierten Plattform und zum Betreiben eines zentralen Brokerservices und eines Extraktionsservices, mit:
- Eine Extraktionseingangsschnittstelle, die zum Einlesen eines Datensatzes von einer lokalen Datenquelle bestimmt ist;
- Eine Recheneinheit, die zum Auswählen von Parametern des eingelesenen Datensatzes zur Erzeugung einer Extraktionsnachricht bestimmt ist;
- Eine Extraktionsausgangsschnittstelle, die zum Versenden der Extraktionsnachricht an einen zentralen Brokerservice bestimmt ist;
- Einem lokalen Repository, das zur lokalen Speicherung der erzeugten Datensätze und zur Versendung von Metadaten zu den erzeugten Datensätzen mit einem Link auf die Datensätze an eine zentrale Registry bestimmt ist;
- Eine Brokereingangsschnittstelle, die zum Empfangen der Anfrage des Clients dient;
- Einer Brokerextraktionsschnittstelle, die in Datenaustausch steht mit der Extraktionsausgangsschnittstelle und zum Einlesen einer Extraktionsnachricht bestimmt ist;
- Einer Verarbeitungseinheit, die zum Auflösen der empfangenen Anfrage und zum Bestimmen eines Client-Identifikators und zum Ermitteln der Parameter der angefragten Übersichtsnachricht bestimmt ist;
- und wobei die Verarbeitungseinheit weiterhin zum Zugriff auf eine Datenbank bestimmt ist, um diejenigen Extraktionsnachrichten zu aggregieren, die den ermittelten Parametern entsprechen;
- und wobei die Verarbeitungseinheit weiterhin zum Zugriff auf die zentrale Registry bestimmt ist, um auf Basis des bestimmten Client-Identifikators eine existierende Berechtigung des Clients zum Zugriff auf das jeweilige Dokument zu überprüfen, in dem der jeweilige Parameter aller aggregierten Extraktionsnachrichten enthalten ist und
- wobei die Verarbeitungseinheit bei existierender Berechtigung zum Einfügen des jeweiligen Parameters in die Übersichtsnachricht und zum Erstellen der Übersichtsnachricht bestimmt ist.

Gemäß einem weiteren Aspekt betrifft die vorliegende Erfindung ein dem vorstehend beschriebenen System entsprechendes Verfahren. Das Verfahren wird verteilt ausgeführt, sowohl lokal auf dem Extraktionsknoten und zentral auf dem Brokerknoten. Das Verfahren dient somit zum Betreiben eines Extraktionsservices auf einem Extraktionsknoten und eines Brokerservices auf einem Brokerknoten zum Erzeugen einer (elektronischen) Extraktionsnachricht für einen Patienten auf Basis einer standardisierten Plattform, umfassend folgende Verfahrensschritte:
1. Einlesen eines Datensatzes von einer lokalen Datenquelle;
2. Auswählen von Parametern des eingelesenen Datensatzes zum Erzeugen einer Extraktionsnachricht;
3. Versenden der Extraktionsnachricht an einen zentralen Brokerservice;
4. Empfangen der Anfrage des Clients;
5. Auflösen der empfangenen Anfrage und Bestimmen eines Client-Identifikators und Ermitteln von Parametern der angefragten Übersichtsnachricht;
6. Zugriff auf eine Datenbank, um diejenigen Extraktionsnachrichten zu aggregieren, die den ermittelten Parametern entsprechen;
7. Zugriff auf eine zentrale Registry, um auf Basis des bestimmten Client-Identifikators eine existierende Berechtigung des Clients zum Zugriff auf das jeweilige Dokument zu überprüfen, in dem der jeweilige Parameter aller aggregierten Extraktionsnachrichten enthalten ist und bejahendenfalls (also falls die Berechtigung vorhanden ist):
8. Einfügen des jeweiligen Parameters in die Übersichtsnachricht und iteratives
9. Erstellen der Übersichtsnachricht.

Dabei werden die Schritte 1 bis 3 auf dem Extraktionsknoten und die restlichen Schritte (4 bis 9) auf dem Brokerknoten oder zentral auf verteilten Cloud-basierten Netzwerkknoten ausgeführt.

Ein wichtiger Vorteil der vorstehend beschriebenen Lösung der Aufgabe ist darin zu sehen, dass die Rechenlast zum Bereitstellen der Übersichtsnachricht als Patient-Summary in das Netzwerk, auf einen Server bzw. in die Cloud verlagert werden kann und nicht lokal auf dem anfragenden Client liegt. Des Weiteren kann nahtlos auf bestehende Standards aufgesetzt werden und die Sicherheitsbestimmungen von schützenswerten, sensiblen Daten können im Gesundheitswesen (PHI-Daten, protected health data) eingehalten werden. Diese sehen es unter anderem vor, dass die lokale in einer Einrichtung (Klinik) erfassten medizinischen Daten eines Patienten nur in dieser Einrichtung gespeichert und nicht extern verfügbar gemacht werden dürfen. Damit können im Stand der Technik bekannte Verfahren, die z.B. mit Webcrawlern arbeiten, nicht verwendet werden.

Vorstehend wurde die Lösung der Aufgabe anhand des Verfahrens, anhand von spezifischen Knoten und eines Systems beschrieben.

Dabei erwähnte Merkmale, Vorteile oder alternative Ausführungsformen sind ebenso auch auf die jeweils anderen beanspruchten Gegenstände zu übertragen und umgekehrt. Mit anderen Worten können auch die gegenständlichen Ansprüche (die beispielsweise auf ein System, einen Konten oder auf ein Computerprogrammprodukt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Eine weitere Aufgabenlösung sieht ein Computerprogramm vor, mit Computerprogrammcode zur Durchführung aller Verfahrensschritte der oben näher beschriebenen Verfahren (auf den unterschiedlichen Knoten), wenn das Computerprogramm auf einem Computer bzw. auf dem jeweiligen Knoten ausgeführt wird. Dabei ist es auch möglich, dass das Computerprogramm auf einem von einem Computer lesbaren Medium gespeichert ist.

Eine weitere Aufgabenlösung sieht ein Computerprogrammprodukt vor, das direkt in den Speicher eines digitalen Computers geladen werden kann und Computerprogrammcode zur Ausführung eines der vorstehend beschriebenen umfasst, wenn das Computerprogrammprodukt auf einem Computer ausgeführt wird.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: eine schematische Übersichtsdarstellung eines Systems zur zentralen Erstellung einer Übersichtsnachricht gemäß einer bevorzugten Ausführungsform der Erfindung;
- Fig. 2: ein Blockschaltbild eines Knotens, der einen lokalen Extraktionsservice gemäß einer bevorzugten Ausführungsform der Erfindung implementiert;
- Fig. 3: ein Blockschaltbild eines Knotens, der einen zentralen Brokerservice gemäß einer bevorzugten Ausführungsform der Erfindung implementiert;
- Fig. 4: ein Ablaufdiagramm eines Verfahrens zur Ausführung auf einem Extraktionsknoten;
- Fig. 5: ein Ablaufdiagramm eines Verfahrens zur Ausführung auf einem Brokerknoten; und
- Fig. 6: ein Sequenz-Diagramm zum Nachrichtenaustausch zwischen beteiligten Knoten.

Die Erfindung betrifft das automatische Erzeugen eines Übersichtdokumentes, das im Folgenden als Übersichtsnachricht und in den Figuren mit dem Bezugszeichen uen gekennzeichnet wird.

Die Übersichtsnachricht uen soll dazu dienen, einem Anwender (Arzt oder der Patient selbst oder andere Beteiligte), der an einem als Client CL ausgebildeten Rechnerknoten arbeitet, einen Überblick über bereits vorliegende Gesundheitsdaten des Patienten zu geben. Die Zusammenstellung der Übersichtsnachricht uen soll konfigurierbar sein. Insbesondere soll - je nach Anwendungsfall - bestimmbar sein, welche Parameter die Übersichtsnachricht uen umfassen soll. In einem ersten Beispiel soll die Übersichtsnachricht uen für Patienten "Huber" das Gewicht und die Blutgruppe umfassen, während in einem zweiten Beispiel für denselben Patienten seine Allergien und ausgeführten OP-Befunde und Berichte inkludiert sein sollen.

Gemäß einer bevorzugten Ausführungsform der Erfindung wird auch die Rolle des anfragenden Clients CL und der situative Anwendungsfall für die Anfrage an (der Anfragekontext) bei der Erzeugung der Übersichtsnachricht uen berücksichtigt. Dies hat folgenden Hintergrund: In medizinischen Berechtigungssystemen gibt es meist Rollen, die einem Anwender/Client zugewiesen sind und die mit Rechten verknüpft sind. So darf z.B. ein Benutzer in der Rolle "Arzt" radiologische Befunde und Entlassungsbriefe einsehen, während ein Benutzer in der Rolle "Pfleger" nur Entlassungsbriefe sehen darf. Damit automatisch geprüft werden kann, ob der jeweilige Benutzer zugriffsberechtigt für das Dokument ist, aus dem die Informationen bzw. Parameter extrahiert wurden, werden beim Aufruf die Rechte und Rollen des Benutzers erfasst. Ebenso wird der Anfragekontext erfasst. Dieser repräsentiert z.B. zu welchem Zweck die Übersichtsnachricht uen generiert werden soll (z.B. Studie, Diagnose etc.).

Zum Erstellen der Übersichtsnachricht uen müssen diverse rechtliche Bedingungen eingehalten werden. So muss beispielsweise sichergestellt werden, dass patienten-spezifische Daten nicht die Sphäre der klinischen Einrichtung verlassen, auf der sie erfasst worden sind. Sie müssen somit lokal gespeichert werden.

Um diese rechtlichen Bedingungen und weitere Anforderungen einhalten zu können, wird vorzugsweise auf eine standardisierte Plattform zum Nachrichtenaustausch zurückgegriffen. Dafür gibt es grundsätzlich mehrere Optionen. Ein bevorzugter Standard ist ein IHE-Standard, insbesondere eine IHE-XDS Plattform.

Nach dem IHE-XDS Standard sind drei Akteure vorgesehen:
- eine lokale Datenquelle XDS-S, auf der die Daten erzeugt werden, z.B. ein MRT-Gerät oder eine andere bildgebende Einrichtung oder ein Blutdruckmessgerät etc. Die Datenquelle XDS-S erzeugt Datensätze ds;
- Ein lokaler Speicher, der als XDS-Repository XDS-REP ausgebildet sein kann. Er dient zur persistenten Speicherung der Binärdaten ds und zur Weiterleitung von Metadaten an eine zentrale Registry XDS-REG;
- Die zentrale Registry XDS-REG. Sie dient zur Speicherung der Metadaten zu den auf der lokalen Datenquelle XDS-S erfassten Datensätzen ds mit einem Zeiger auf den Speicherort derselben, also auf das lokale Repository XDS-REP. Die Datensätze ds werden nicht auf der zentralen Registry XDS-REG gespeichert, sondern dort nur registriert.

Diese standardkonforme Architektur wird erfindungsgemäß erweitert durch zwei interagierende Knoten:
- Einen lokalen Extraktionsknoten OES und
- Einen zentralen Brokerknoten OBS.

Der Extraktionsknoten OES ist vorzugsweise ausgebildet, um einen Extraktionsservice (oder -Dienst) auszuführen. Der Brokerknoten OBS ist vorzugsweise ausgebildet, um einen Brokerservice auszuführen.

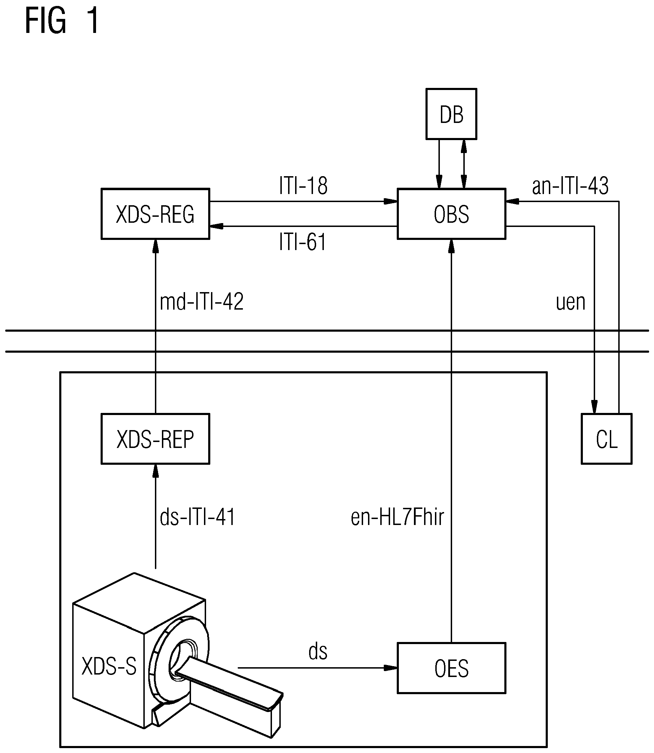

Das Zusammenspiel dieser Akteure wird im Folgenden unter Bezugnahme auf Fig. 1 näher erläutert.

Der Extraktionsservice dient zum Empfang der erfassten Datensätze ds und extrahiert daraus nach vorkonfigurierbaren Regeln bestimmte Parameter von Interesse, die grundsätzlich für Übersichtsnachrichten zur Verfügung stehen sollen. Der Extraktionsservice erzeugt somit aus dem jeweiligen Datensatz ds ein Exzerpt und strukturiert dies in Form einer Extraktionsnachricht en und sendet diese an den zentralen Brokerservice. Zum Transfer kann eine HL7 FHIR Transaktion verwendet werden (FHIR: Fast Healthcare Interoperability Resources).
Die Extraktionsnachricht en umfasst zumindest folgende Einträge:
- Einen Patienten-Identifikator; dies ist eine eineindeutige Kennzahl, die den Patienten identifiziert
- Einen Dokumenten-Identifikator; dies ist ebenfalls eine eineindeutige Kennzahl, die das Dokument identifiziert bzw. dessen Speicherort referenziert, aus dem die extrahierten Parameter erfasst worden sind und
- Die jeweiligen Parameter.

Der zentrale Brokerservice dient zum Empfang der Extraktionsnachricht en. Weiterhin ist er Empfängerknoten einer Anfrage des Clients CL. Der Client CL möchte z.B. in seiner Funktion als Anästhesist eine Übersicht für einen bestimmten Patienten Huber erhalten zur Planung der anstehenden Anästhesie anlässlich eines operativen Eingriffs. Dazu interessieren ihn spezielle Parameter, wie Allergien, Gewicht und Alter des Patienten und Narkosegase, die bei bisherigen Operationen zum Einsatz kamen. Er erstellt dazu eine konfigurierte Anfrage an, die Parameter Allergien, Gewicht, Alter und Narkosegase umfassen. Diese Anfrage wird über eine Transaktion, bevorzugt eine HL7 FHIR Transaktion an den zentralen Brokerservice übermittelt.

Der zentrale Brokerservice löst nach Erhalt der Anfrage an diese auf und extrahiert bzw. bestimmt einen Client-Identifikator, der den anfragenden Client CL identifiziert. Dabei kann sowohl eine Rolle der anfragenden Person (Eigenschaft als Arzt, insbesondere Anästhesist) als auch der Kontext der Anfrage (für eine anstehende Anästhesie) berücksichtigt werden. Der Client-Identifikator umfasst dazu kennzeichnende Elemente. Des Weiteren ermittelt der zentrale Brokerservice die gewünschten Parameter für die angefragte Übersichtsnachricht uen für den jeweiligen Patienten. Üblicherweise ist die Anfrage an patienten-spezifisch. Es können allerdings auch mehrere Anfragen an kombiniert werden zu einer komplexeren Query (z.B. "Übersicht zu allen Patienten mit OP-Termin morgen in Hinblick auf die Parameter Allergien, Gewicht, Alter und Narkosegase"), die dann patienten-übergreifend ist.

Mit den ermittelten Parametern führt der zentrale Brokerservice einen Zugriff auf eine Datenbank DB aus, um die Einträge bzw. die Extraktionsnachrichten zu finden, die die gewünschten Parameter umfassen. Der Datenbankzugriff ermittelt somit eine Menge von Extraktionsnachrichten, die - im obigen Beispiel - Angaben zu Allergien, Gewicht, Alter und Narkosegase des Patienten Huber.

Da der anfragende Client Cl aber nicht immer auf alle Dokumente zugriffsberechtigt ist, ist es notwendig, dass der zentrale Brokerservice weitere Transaktionen zur Überprüfung der jeweiligen Berechtigungen des Client Cl auf die Dokumente ausführt. Dazu greift er vorzugweise auf die zentrale Registry XDS-REG zu. Dies kann über eine ITI-18 bzw. ITI-61 Transkation ausführen. In einer vorteilhaften alternativen Ausführungsform der Erfindung kann der Zugriff auf die zentrale Registry XDS-REG auch indirekt ausgeführt werden, vermittelt durch ein Berechtigungssystem (access control system - ACS).

Der zentrale Brokerservice oder das Berechtigungssystem prüft nun für die Liste mit den passenden Extraktionsnachrichten iterativ für jede der Extraktionsnachrichten, welche Parameter sie umfasst und aus welchem Dokument der jeweilige Parameter stammt, um zu ermitteln, ob der anfragende Client CL zum Zugriff auf das jeweilige Dokument berechtigt ist. Nur falls eine Berechtigung festgestellt werden konnte, wird der jeweilige Parameter in die zu erzeugende Übersichtsnachricht uen inkludiert. Andernfalls (ohne Berechtigung) wird der Parameter nicht in die Übersichtsnachricht uen aufgenommen.

In mehrfachen Iterationen kann letztendlich die Übersichtsnachricht uen sozusagen on-the-fly erzeugt und als strukturiertes Dokument an den anfragenden Client CL ausgeliefert werden. Zur Kommunikation zwischen dem Client CL und dem zentralen Brokerservice kann eine ITI-43 oder eine HL7 FHIR Transaktion verwendet werden.

Wie in Fig. 1 gezeigt, sind die Datenquelle XDS-S, der Extraktionsservice und das Speichersystem XDS-REP lokal in einer klinischen Einrichtung positioniert. Die korrespondierenden Knoten XDS-S, OES, XDS-REP kommunizieren über standardisierte Protokolle, wie z.B. über ITI-41. Dies ist eine Transaktion mit Inhalt (payload) einer sogenannten "provide and register document set-b" Nachricht. Der payload der Nachricht kann Metadaten md zu dem erfassten Datensatz enthalten mit den Zugriffsberechtigungen und weiteren Metadaten, wie z.B. einem Zeitstempel, der jeweiligen Datenquelle XDS-S etc.

Die Metadaten md werden mit einem Link zum Speicherort der Daten ds an eine zentrale Registry XDS-REG zur Registrierung gesendet. Die Registry XDS-REG ist als zentrale Instanz vorgesehen und kann sozusagen in die Cloud verlagert werden. Als zentrale Instanz ist zusätzlich der Brokerservice auf dem Brokerknoten OBS vorgesehen, der ebenfalls über eine geeignete Netzwerkanbindung in Datenaustausch mit den anderen Akteuren steht.

Die Kommunikation zwischen dem lokalen Repository XDS-REP und der zentralen Registry XDS-REG kann z.B. über eine ITI-42 Transaktion erfolgen.

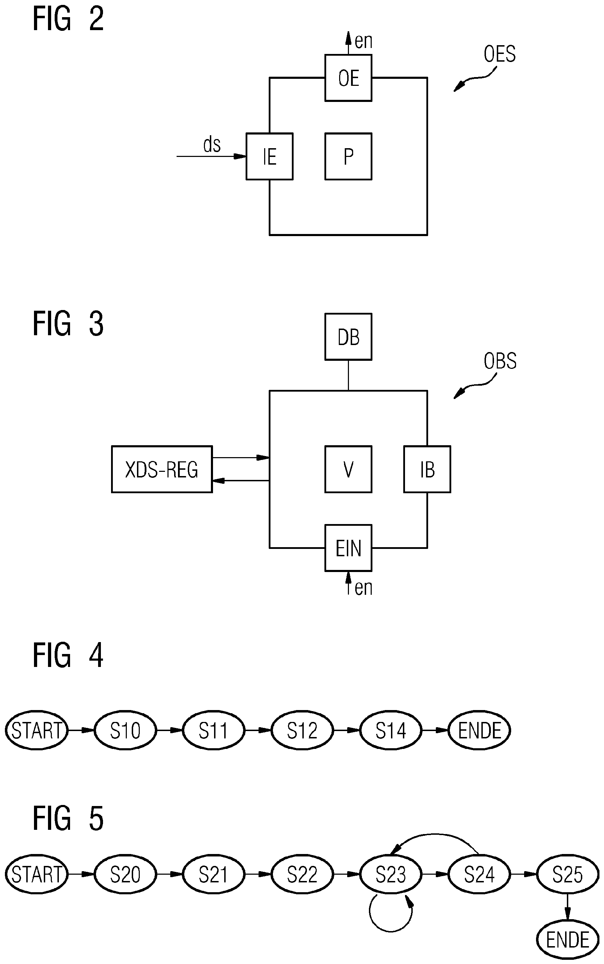

Fig. 2 zeigt auf schematische Weise die Architektur des Extraktionsknotens OES auf dem der Extraktionsservice implementiert ist. Er umfasst eine Extraktionseingangsschnittstelle IE zum Empfang der auf der Datenquelle XDS-S erfassten Datensätze ds und eine Recheneinheit P zur Extraktion von relevanten Parametern aus den Datensätzen ds. Die Recheneinheit P berechnet die Extraktionsnachricht en und sendet diese über die Extraktionsausgangsschnittstelle OE an den zentralen Brokerservice. Fakultativ kann der Extraktionsservice noch weitere Bestandteile aufweisen, wie z.B. einen Cache oder einen Speicher zur (Zwischen-) Speicherung der erzeugten Extraktionsnachricht en.

In Fig. 3 ist auf schematische Weise die Architektur des Brokerknotens OBS, auf dem der zentrale Brokerservice implementiert ist, dargestellt. Er umfasst eine Brokerextraktionsschnittstelle EIN zum Empfang der Extraktionsnachricht en und eine weitere Schnittstelle zur Kommunikation mit der Registry XDS-REG sowie eine weitere Brokereingangsschnittstelle IB, die zum Empfangen der Anfrage an des Clients CL dient. Eine Verarbeitungseinheit V dient zum Auflösen der empfangenen Anfrage an und zum Bestimmen eines Client-Identifikators und zum Ermitteln der Parameter der angefragten Übersichtsnachricht uen für den Patienten. Vorzugsweise wird auch eine Patienten-ID in der Anfrage an übermittelt und auf dem Brokerserviceknoten OBS herausgelöst.

Mit Bezugnahme auf Fig. 4 wird im Folgenden ein Ablauf des Verfahrens beschrieben, das auf dem Extraktionsknoten OES ausgeführt wird.

Nach dem Start des Verfahrens wird in Schritt S10 der Datensatz ds von der Quelle XDS-S empfangen und in Schritt S11 werden vorkonfigurierte Parameter mit deren Werten ausgewählt. Hier werden z.B. relevante Parameter ausgewählt (z.B. Gewicht, Alter, Allergie, Narkosegaszusammensetzung) und deren Werte bestimmt, um daraus eine Extraktionsnachricht en in Schritt S12 zu erzeugen. Die Extraktionsnachricht en beinhaltet dann z.B. die Informationen <Code für Gewicht>, 75, <optional Einheit> kg, <dokumentenID>, <patientID>. Die extrahierten Informationen werden in Schritt S14 an den Brokerservice versendet, welcher die Informationen speichert, insbesondere in der Datenbank DB. Wichtig ist hier zu betonen, dass es sich bei den gespeicherten Daten nicht um die originalen, sensiblen medizinischen Daten handelt, sondern nur um Exzerpte bzw. sicherheitsunkritische Auszüge derselben. Auf der entstehenden Datenansammlung können dann vorteilhafterweise gewisse Operationen implementiert werden (wie z.B. die automatische Erstellung einer Übersichtsnachricht bzw. einer Patient-Summary oder auch Abfragen von konkreten Werten und/oder weitere (z.B. statistische) Auswertungen der Daten). Das Verfahren kann im PUSH-Betrieb betrieben werden, so dass neu erzeugte Dokumente bzw. Datensätze immer in den Extraktionsknoten verschoben werden. Alternativ können die erzeugten Datensätze ds auch zu bestimmten Zeiten oder nach konfigurierbaren Ereignissen dem Extraktionsservice zugeführt werden. Alternativ dazu wird das Verfahren im PULL-Modus betrieben, so dass der Extraktionsknoten das Zusenden von Datensätzen ds triggert und anfordert.

Unter Bezugnahme auf Fig. 5 wird im Folgenden ein Ablauf des Verfahrens beschrieben, das auf dem Brokerknoten OBS ausgeführt wird.

Nach Start des Verfahrens wird in Schritt S20 die Anfrage an des Clients CL empfangen. Diese wird in Schritt S 21 aufgelöst um den Client-Identifikator zu bestimmen und die gewünschten Parameter der Anfrage bzw. der angefragten Übersichtsnachricht uen zu ermitteln. Daraufhin kann in Schritt S22 ein Zugriff auf die angeschlossene Datenbank DB erfolgen, um diejenigen Extraktionsnachrichten en zu suchen und zu aggregieren, die den ermittelten Parametern entsprechen. Dabei werden also vorzugsweise all diejenigen Extraktionsnachrichten en ermittelt, die die Parameter umfassen. Als Zwischenergebnis wird eine Liste mit ermittelten Extraktionsnachrichten en generiert. Jede Extraktionsnachricht en umfasst üblicherweise mehrere Parameter. Dabei handelt es sich deshalb nur um ein Zwischenergebnis, da für jeden der Parameter noch geprüft werden muss, ob der anfragende Client CL in seiner Rolle und in seinem Anfragekontext auch zum Zugriff auf die Dokumente berechtigt ist, aus denen der jeweilige Parameter extrahiert worden ist. Ein Parameter kann auch in mehreren Dokumenten bzw. Datensätzen ds enthalten sein. Für den Fachmann ist offensichtlich, dass diese Aufgabe mit einer hohen Rechenlast einhergeht. Die Rolle und der Anfragekontext werden bei Erzeugung der Anfrage vom Client CL erfasst und in einem Datenpaket mit der Anfrage-Query in der Anfragenachricht an an den Brokerservice gesendet.

Deshalb ist ein wichtiger Vorteil der erfindungsgemäß Lösung darin zu sehen, dass diese rechenlast-intensiven Operationen vollständig in die Cloud, also auf den zentralen Brokerservice bzw. den Brokerknoten OBS ausgelagert werden können und nicht auf den lokalen Knoten oder auf dem Client CL ausgeführt werden müssen. Insbesondere muss kein Datenbankzugriff und keine Suche auf den lokalen Knoten ausgeführt werden, was das hier vorgeschlagene Vorgehen sehr effizient macht.

In Schritt S23 erfolgt - auch wieder ausgehend von dem Brokerservice - ein Zugriff auf die zentrale Registry XDS-REG, um auf Basis des bestimmten Client-Identifikators eine existierende Berechtigung des Clients CL zum Zugriff auf das jeweilige Dokument / Datensatz ds zu überprüfen, in dem der jeweilige Parameter aller aggregierten Extraktionsnachrichten en enthalten ist. Bejahendenfalls (also falls die Berechtigung vorhanden ist) wird der jeweilige Parameter in Schritt S24 in die Übersichtsnachricht uen eingefügt. Diese Schritte werden iterativ für alle Berechtigungen aller Datensätze ds zu allen Parametern aller Extraktionsnachrichten en des Zwischenergebnisses wiederholt, bis für alle Elemente geklärt ist, ob die Zugriffsberechtigung existiert oder nicht. Daraufhin kann letztendlich in Schritt S25 die Übersichtsnachricht uen erstellt werden. Sie kann über eine standardisierte Transaktion an den Client CL ausgeliefert werden. Danach kann das Verfahren enden.

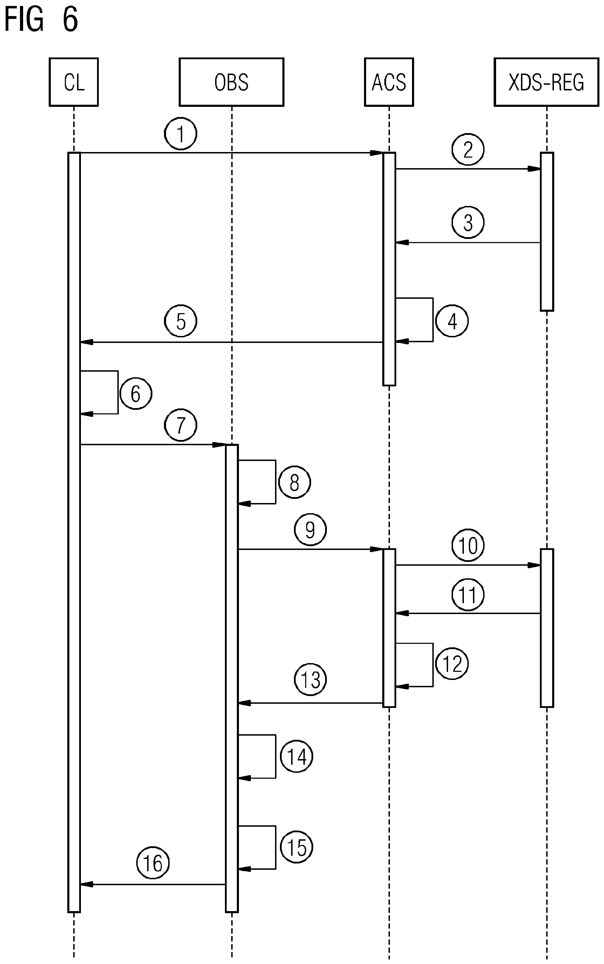

Fig. 6 zeigt ein UML-Diagramm für den Datenaustausch zwischen beteiligten Knoten gemäß einer bevorzugten Implementierung. Dabei ist auch ein Zugriffskontrollknoten vorgesehen auf dem ein Zugriffskontrollservice ACS (access control system) implementiert ist. Er wird im Folgenden deshalb auch kurz als ACS Knoten bezeichnet und trägt das Bezugszeichen ACS. In alternativen Ausführungsformen der Erfindung wird die Funktionalität des ACS Knotens in einen anderen zentralen Knoten integriert, insbesondere in die zentrale Registry XDS-REG.

Der in Fig. 6 repräsentierte Client CL kann ein XDS Empfänger sein (also eine Datensenke, z.B. ein Arzt, der Daten erfragen möchte) oder ein XDS Sender (und damit eine Datenquelle), der selbst Daten generiert. Mit Transkation 1 kann der Client CL auf den ACS-Knoten zugreifen, der daraufhin den Datensatz ds mit Transaktionen 2, 3 in der XDS-Registry XDS-REG registriert. Der ACS-Knoten kann dann noch Filteroperationen vornehmen, z.B. um erfasste Policies und weitere Berechtigungen umzusetzen. Mit Transaktion 5 kann ein Ergebnis an den Client Cl ausgegeben werden.

Der Client CL stellt mit der in Fig. 6 repräsentierten Transaktion 7 eine Anfrage an an den Brokerservice, wie in Fig. 6 gezeigt. Nachdem der Brokerservice die notwendigen Daten aus der Anfrage an herausgelöst hat, kann dieser mit Transkation 8 eine Suche nach - zur Anfrage an und zum Client CL korrespondierenden - Extraktionsnachrichten en in der Datenbank DB ausführen. Dann erfolgt in Transaktion 9 ein Zugriff auf den ACS-Knoten (oder direkt auf die Registry XDS-REG), der mit Transkation 10 auf die Registry XDS-REG zugreift, um die Zugriffsberechtigungen zu ermitteln und mit Transkation 11 auf dem ACS-Knoten bereitzustellen. Dort können mit Transkation 12 Filteroperationen ausgeführt werden. Es können auch mehrere Filteroperationen 12 hintereinander ausgeführt werden, um beispielsweise hinterlegte Policies zu erfüllen (die z.B. regeln können, dass bestimmte Dokumente / Datensätze ds nicht an bestimmte Clients CL herausgegeben werden dürfen oder nur in vordefinierten Zeiträumen). Es kann auch sein, dass die Weitergabe von bestimmten Datensätzen ds mit einem Einverständnis des Patienten verknüpft ist, das als Flag in einem Register gesetzt (bzw. nicht gesetzt) ist. Entsprechend wird eine Filterung ausgeführt für diejenigen Datensätze, für die das Einverständnis vorliegt. Die Anwendung von weiteren Steuer- und Regelungsmechanismen liegt ebenso im Rahmen der Erfindung. Die Filteroperation, die der ACS-Knoten ausführt, können es erforderlich machen, noch auf weitere Knoten zugreifen zu müssen (etwa zum Datenabgleich). Diese weiteren Knoten sind in Fig. 6 allerdings - zum Zwecke der besseren Übersichtlichkeit - nicht dargestellt. Mit Transkation 13 kann das Zwischenergebnis an den Brokerservice weitergereicht werden. Dieser kann mit der Operation 14 noch weitere Prozesse ausführen und z.B. bestimmte Extraktionsnachrichten en aus der Übersichtsnachricht uen entfernen, für die keine Berechtigung vorliegt. Mit Transkation 15 wird das Ergebnis mit der Übersichtsnachricht uen spezifisch für den anfragenden Client CL zusammengestellt und mit Transkation 16 an diesen ausgegeben. Bei dem Ergebnis handelt es sich vorzugsweise um ein Dokument im CDA-Format (Clinical Document Architecture des HL7 Standards).

Im Folgenden sei ein Beispiel für eine Übersichtsnachricht uen gegeben, wobei allerdings die Dokumenten-Identifikator nicht explizit genannt ist, aber entsprechend aufgebaut ist wie der Patienten-Identifikator <Patient id>:

```
 {
   "resourceType":"Observation",
   "contained":[
      {
         "resourceType":"Patient",
         "id":"1"
         "identifier":[
             {
                "use":"usual",
                "system":"1.1.1",
                "value":"ee499dd1-06f5-4335-9304-67536aa5c6ad"
             }
         ]
      }
   ],
   "status":"preliminary",
   "category":[
      {
         "coding":[
             {
                "system":"http://hl7.org/fhir/observation-
 category",
                "code":"vital-signs",
                "display":"Vital Signs"
             }
         ]
      }
   ],
   "code":{
      "coding":[
          {
             "system":"http://loinc.org",
             "code":"3141-9",
             "display":"Weight Measured"
          }
      ]
      "text":"LOINC Body weight"
   },
   "subject":{
      "reference":"#1"
   },
   "effectivePeriod":{
      "start":"2017-02-01T13:33:17+01:00",
      "end":"2017-02-01T13:33:17+01:00"
   },
   "valueQuantity":{
      "value":80.0,
      "unit":"kg"
      "system":"http://unitsofmeasure.org",
      "code":"[kg]"
   },
   "comment":""
 }
```

Zusammenfassend lässt festhalten dass es mit dem erfindungsgemäßen Vorschlag möglich wird, medizinische Datensätze ds auf konfigurierte Weise zu indizieren und in indizierter Form abzulegen, um eine sehr effiziente Suche in den umfangreichen Datensätzen auf einem dedizierten leistungsstarken Server zu ermöglichen, um eine Übersichtsdatei oder eine Übersichtsnachricht uen generieren zu können und dabei sowohl die Sicherheitsbestimmungen einhalten und auf bestehende Standards aufsetzen zu können, ohne dass Änderungen an dem bestehenden Verwaltungsservice oder an der der bestehenden Hardware vorgenommen werden müssen.

Eine grundlegende Idee der vorliegenden Erfindung besteht darin, ein System bereitzustellen, mit dem eine Übersichtsnachricht automatisch erstellt werden kann ohne, dass an der bestehenden Infrastruktur des medizinischen IT-Systems Änderungen ausgeführt werden müssen. In der Übersichtsnachricht uen können vorkonfigurierten Datenauswertungen repräsentiert sein, wie eine Patienten-Summary oder andere Auswertungen, wie z.B. die Ausgabe eines Warnsignals bei kritischen Veränderungen an den Datensätzen. Durch geschickte Abfolge und Aneinanderreihung von Transaktionen kann die Übersichtsnachricht uen so berechnet werden, dass sie nur diejenigen Bestandteile enthält, für die das Berechtigungssystem ACS, XDS-REG Zugriffsberechtigungen validiert hat. Das ist einer der Kernpunkte der vorliegenden Anmeldung, dass die in der Übersichtsnachricht uen enthaltenen Informationen, die sogenannten Observationen (bzw. Beobachtungen), nicht nur extrahiert und abgelegt werden, sondern zusätzlich mit bestehenden dokumentenbasierten Berechtigungssystemen verarbeitet werden können. Damit können die Kosten gesenkt werden, da keine neue Hardware finanziert werden muss. Es kann auf bestehende Systeme mit üblichen Standards aufgesetzt werden.

Mit dieser Lösung kann der technische Vorteil erzielt werden, dass die rechenlastintensiven Berechnungen alle in die Cloud verlagert werden können. In der Cloud kann dafür ein dedizierter hoch-performanter Dienstknoten bereitgestellt werden. Der Erzeugungsprozess zur Erzeugung der Übersichtsnachricht kann vollständig auf der Seite der Datenerzeugung und nicht auf der Seite der Datenkonsumierung, also nicht client-seitig, ausgeführt werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Für einen Fachmann ist es insbesondere offensichtlich, dass die Erfindung nicht nur für IHE-XDS Systeme angewendet werden kann, sondern auch für andere Standards. Vorzugsweise sind die lokalen Knoten getrennt von den zentralen Knoten. Es ist jedoch in speziellen Ausführungsformen der Erfindung, etwa, wenn die Seite der Datenerzeugung über sehr viel Ressourcen verfügt, auch möglich, dass der Brokerservice auf demselben Knoten implementiert ist, wie der Extraktionsservice. Üblicherweise sind jedoch der Knoten OBS des Brokerservices und der Knoten OES des Extraktionsservice auf mehrere physikalische Produkte verteilt realisiert.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

## Patentansprüche

1. Verfahren zum Betreiben eines zentralen Brokerservices zum Bedienen einer Anfrage (an) eines Clients (CL) für eine Übersichtsnachricht (uen) für einen Patienten auf Basis einer standardisierten Plattform, umfassend folgende Verfahrensschritte:
- Empfangen (S20) der Anfrage (an) des Clients (CL);
- Auflösen (S21) der empfangenen Anfrage (an) und Bestimmen eines Client-Identifikators und Ermitteln von Parametern der angefragten Übersichtsnachricht (uen);
- Zugriff (S22) auf eine Datenbank (DB), um diejenigen Extraktionsnachrichten (en) zu aggregieren, die den ermittelten Parametern entsprechen;
- Zugriff (S23) auf eine zentrale Registry (XDS-REG), um auf Basis des bestimmten Client-Identifikators eine existierende Berechtigung des Clients (CL) zum Zugriff auf das jeweilige Dokument zu überprüfen, in dem der jeweilige Parameter aller aggregierten Extraktionsnachrichten (en) enthalten ist und bejahendenfalls: Ausführen der folgenden Verfahrensschritte:
- Einfügen (S24) des jeweiligen Parameters in die Übersichtsnachricht (uen) zum
- Erstellen (S25) der Übersichtsnachricht (uen).

2. Verfahren nach Anspruch 1, bei dem die Übersichtsnachricht (uen) dynamisch und nur bei Bedarf auf Anfrage des Clients (CL) und insbesondere während des Zugriffs (S22) auf die Datenbank (DB) erzeugt wird.

3. Verfahren nach einem der vorangehenden Ansprüche, bei dem die Übersichtsnachricht (uen) als vollstrukturiertes Dokument, insbesondere nach einem CDA-Standard formatiert, ausgeliefert wird.

4. Verfahren nach einem der vorangehenden Ansprüche, bei dem eine Berechtigung des Clients (CL) auf einer Berechtigungsregel basiert, wobei die Berechtigungsregel in der zentralen Registry (XDS-REG) hinterlegt ist, und wobei die Berechtigungsregel auch während der Ausführung des Verfahrens veränderlich ist.

5. Verfahren nach einem der vorangehenden Ansprüche, bei dem beim Erzeugen der Übersichtsnachricht (uen) eine Berechtigung des anfragenden Clients (CL) zum Zugriff auf Datensätze und/oder Kontextparameter der Anfrage (an) berücksichtigt werden.

6. Verfahren nach einem der vorangehenden Ansprüche, bei dem zum Erzeugen der Übersichtsnachricht (uen) deren angefragte Parameter nach konfigurierbaren Filterkriterien gefiltert werden.

7. Verfahren nach einem der vorangehenden Ansprüche, bei dem die Übersichtsnachricht (uen) einen Link auf das jeweilige Dokument umfasst, in dem der/die angefragte(n) Parameter enthalten ist/sind.

8. Verfahren nach einem der vorangehenden Ansprüche, bei dem die Übersichtsnachricht (uen) nicht in der Registry (XDS-REG) und insbesondere auf keinem Knoten gespeichert, sondern nur dem Client (CL) zur Verfügung gestellt wird.

9. Verfahren nach einem der vorangehenden Ansprüche, bei dem alle Berechnungen zur Erzeugung der Übersichtsnachricht (uen) vollständig durch den zentralen Brokerservice auf einem zentralen Brokerknoten (OBS) ausgeführt werden.

10. Verfahren nach einem der vorangehenden Ansprüche, bei dem die standardisierte Plattform eine IHE-XDS Plattform ist.

11. Verfahren zum Betreiben eines Extraktionsservices zum Erzeugen einer Extraktionsnachricht (en) für einen Patienten auf Basis einer standardisierten Plattform, umfassend folgende Verfahrensschritte:
- Einlesen (S10) eines Datensatzes (ds) von einer lokalen Datenquelle (XDS-S);
- Auswählen (S11) von Parametern des eingelesenen Datensatzes (ds) zum Erzeugen (S12) einer Extraktionsnachricht (en);
- Versenden (S14) der Extraktionsnachricht (en) an einen zentralen Brokerservice.

12. Verfahren nach dem unmittelbar vorangehenden Anspruch, bei dem die ausgewählten Parameter aus dem eingelesenen Datensatz (ds) vorkonfigurierbar sind.

13. Verfahren nach einem der vorstehenden Ansprüche 11 oder 12, bei dem die Extraktionsnachricht (en) zumindest umfasst:
- Einen Patienten-Identifikator;
- einen Dokumenten-Identifikator, der einen Speicherort von Metadaten zu dem eingelesenen Datensatz, aus dem die Daten extrahiert wurden, eindeutig referenziert;
- eine konfigurierbare Auswahl von extrahierten Parametertypen mit deren Werten, wobei ein Code einen Parametertyp eindeutig kennzeichnet.

14. Computerprogrammprodukt, das direkt in den Speicher eines Computers geladen werden kann und Computerprogrammcode zur Ausführung des Verfahrens nach einem der vorstehenden Ansprüche umfasst, wenn das Computerprogrammprodukt auf einem Computer ausgeführt wird.

15. Extraktionsknoten (OES) zum Erzeugen einer Extraktionsnachricht (en) für einen Patienten auf Basis einer standardisierten Plattform, umfassend:
- Eine Extraktionseingangsschnittstelle (IE), die zum Einlesen (S10) eines Datensatzes (ds) von einer lokalen Datenquelle (XDS-S) bestimmt ist;
- Eine Recheneinheit (P), die zum Auswählen (S11) von Parametern des eingelesenen Datensatzes (ds) zur Erzeugung (S12) einer Extraktionsnachricht (en) bestimmt ist;
- Eine Extraktionsausgangsschnittstelle (OE), die zum Versenden (S14) der Extraktionsnachricht (en) an einen zentralen Brokerservice bestimmt ist.

16. Zentraler Brokerknoten (OBS), der zur Ausführung eines Brokerservices dient zum Bedienen einer Anfrage (an) eines Clients (CL) für eine Übersichtsnachricht (uen) für einen Patienten auf Basis einer standardisierten Plattform, umfassend:
- Eine Brokereingangsschnittstelle (IB), die zum Empfangen (S20) der Anfrage (an) des Clients (CL) dient;
- Einer Brokerextraktionsschnittstelle (EIN), die zum Einlesen einer Extraktionsnachricht (en) bestimmt ist;
- Einer Verarbeitungseinheit (V), die zum Auflösen (S21) der empfangenen Anfrage (an) und zum Bestimmen eines Client-Identifikators und zum Ermitteln der Parameter der angefragten Übersichtsnachricht (uen) bestimmt ist;
- und wobei die Verarbeitungseinheit (V) weiterhin zum Zugriff (S22) auf eine Datenbank (DB) bestimmt ist, um diejenigen Extraktionsnachrichten (en) zu aggregieren, die den ermittelten Parametern entsprechen;
- und wobei die Verarbeitungseinheit (V) weiterhin zum Zugriff (S23) auf eine zentrale Registry (XDS-REG) bestimmt ist, um auf Basis des bestimmten Client-Identifikators eine existierende Berechtigung des Clients (CL) zum Zugriff auf das jeweilige Dokument zu überprüfen, in dem der jeweilige Parameter aller aggregierten Extraktionsnachrichten (en) enthalten ist und
- wobei die Verarbeitungseinheit (V) bei existierender Berechtigung zum Einfügen (S24) des jeweiligen Parameters in die Übersichtsnachricht (uen) und zum Erstellen (S25) der Übersichtsnachricht (uen) bestimmt ist.

17. System zum Erzeugen einer Übersichtsnachricht (uen) für einen Patienten auf Basis einer standardisierten Plattform und zum Betreiben eines zentralen Brokerservices und eines Extraktionsservices, mit:
- Eine Extraktionseingangsschnittstelle (IE), die zum Einlesen (S10) eines Datensatzes (ds) von einer lokalen Datenquelle (XDS-S) bestimmt ist;
- Eine Recheneinheit (P), die zum Auswählen (S11) von Parametern des eingelesenen Datensatzes (ds) zur Erzeugung (S12) einer Extraktionsnachricht (en) bestimmt ist;
- Eine Extraktionsausgangsschnittstelle (OE), die zum Versenden (S14) der Extraktionsnachricht (en) an einen zentralen Brokerservice bestimmt ist;
- Einem lokalen Repository (XDS-REP), das zur lokalen Speicherung der erzeugten Datensätze (ds) und zur Versendung von Metadaten zu den erzeugten Datensätzen (ds) mit einem Link auf die Datensätze (ds) an eine zentrale Registry (XDS-REG) bestimmt ist;
- Eine Brokereingangsschnittstelle (IB), die zum Empfangen (S20) der Anfrage (an) des Clients (CL) dient;
- Einer Brokerextraktionsschnittstelle (EIN), die in Datenaustausch steht mit der Extraktionsausgangsschnittstelle (OE) und zum Einlesen einer Extraktionsnachricht (en) bestimmt ist;
- Einer Verarbeitungseinheit (V), die zum Auflösen (S21) der empfangenen Anfrage (an) und zum Bestimmen eines Client-Identifikators und zum Ermitteln der Parameter der angefragten Übersichtsnachricht (uen) bestimmt ist;
- und wobei die Verarbeitungseinheit (V) weiterhin zum Zugriff (S22) auf eine Datenbank (DB) bestimmt ist, um diejenigen Extraktionsnachrichten (en) zu aggregieren, die den ermittelten Parametern entsprechen;
- und wobei die Verarbeitungseinheit (V) weiterhin zum Zugriff (S23) auf die zentrale Registry (XDS-REG) bestimmt ist, um auf Basis des bestimmten Client-Identifikators eine existierende Berechtigung des Clients (CL) zum Zugriff auf das jeweilige Dokument zu überprüfen, in dem der jeweilige Parameter aller aggregierten Extraktionsnachrichten (en) enthalten ist und
- wobei die Verarbeitungseinheit (V) bei existierender Berechtigung zum Einfügen (S24) des jeweiligen Parameters in die Übersichtsnachricht (uen) und zum Erstellen (S25) der Übersichtsnachricht (uen) bestimmt ist.
